# EUROPÄISCHE PATENTSCHRIFT

(11) **EP 2 642 326 B1**
(45) Veröffentlichungstag und Bekanntmachung des Hinweises auf die Patenterteilung: **19.08.2020**
(21) Anmeldenummer: 13001440.0
(22) Anmeldetag: 20.03.2013
(51) Int. Cl.: G02B 21/36, G02B 21/34, G01N 33/53, B01L 9/00, G01N 35/00, G02B 21/26

(54) **Automatische Kalibrierung eines Mikroskop-Scanningsystems**
Automatic calibration of a microscope scanning system
Etalonnage automatique d'un microscope à balayage

(30) Priorität: 20.03.2012 DE 102012005587
(43) Veröffentlichungstag der Anmeldung: 25.09.2013
(73) Patentinhaber: MetaSystems Hard & Software GmbH, 68804 Altlussheim (DE)
(72) Erfinder: Lörch, Thomas, 68804 Altlussheim (DE)
(74) Vertreter: Müller-Boré & Partner Patentanwälte PartG mbB

(56) Entgegenhaltungen:
- US-A- 5 428 690
- US-A- 5 694 212
- US-A1- 2003 027 342
- US-A1- 2005 051 614

## Beschreibung

Die Erfindung betrifft ein Verfahren zur automatischen Kalibrierung von Objektträgern und ein Verfahren zur automatischen Kalibrierung von Objektträgerrahmen sowie einen Objektträger zur Durchführung des Verfahrens Verfahren zur automatischen Kalibrierung von Objektträgerrahmen.

Im Allgemeinen werden Objektträger zur Mikroskopie verwendet. Beispielsweise werden Zell- oder Gewebeproben auf einem Objektträger aufgetragen bzw. dort positioniert. Der Objektträger mit der Zell- oder Gewebeprobe wird dann auf einem Mikroskoptisch eines Mikroskops gelegt, so dass die Zell- oder Gewebeprobe mikroskopiert werden kann.

Weiterhin werden Mikroskop-Scanningsysteme eingesetzt, um Objekte wie Zellen einer Gewebeprobe, welche auf einem Objektträger positioniert sind, automatisch zu finden. Dies können beispielsweise Zellen mit einer bestimmten Eigenschaft (bspw. eine Markierung) sein, die in einem Gewebeschnitt vorliegen, der auf dem Objektträger liegt. Eine Markierung kann beispielsweise eine Einfärbung einer Zelle sein.

Die Mikroskoptische bzw. Scanningtische sind als Messtische mit Koordinatensystemen ausgestattet, so dass beispielsweise von einem Koordinatenursprung des Messtisches und/oder des Objektträgers aus die Position des gefundenen Objekts nachvollziehbar bestimmt werden kann.

In der Regel wird die Position der gefundenen Objekte abgespeichert. Dies kann dazu dienen, nach dem Absuchen eines Präparates (bspw. Gewebeprobe) auf einem Objektträger die gefundenen Objekte (bspw. Zellen) automatisch zu relokalisieren bzw. erneut zu überprüfen, d.h. dem Beobachter nochmals zur visuellen Überprüfung zu präsentieren. Solange die Relokalisation direkt nach dem Absuchen des Präparats stattfindet, d.h. ohne dass das Präparat aus seiner ursprünglichen Position auf dem Scanningtisch entfernt wurde, ist ein absolutes X-Y-Z-Koordinatensystem nicht erforderlich, um eine präzise Relokalisation zu garantieren. In diesem Fall ist ausreichend, die bei der Suche abgespeicherten relativen Koordinaten wieder anzufahren.

In vielen Fällen kann die Relokalisation nicht unmittelbar nach dem Absuchen des Präparats erfolgen. Dies ist insbesondere dann der Fall, wenn die Suche im "Batch"-Betrieb durchgeführt wird. Hierbei wird beispielsweise zuerst eine bestimmte Anzahl von Präparaten auf den Objektträgern - z.B. über Nacht - automatisch abgesucht und erst am nächsten Morgen sollen die gefundenen Objekte überprüft werden.

In einem solchen Betrieb wird es regelmäßig erforderlich sein, die abgesuchten Objektträger mit Hilfe eines automatischen Wechslers/Robotermoduls vom Scanningtisch zu entfernen und einen neuen Satz von Objektträgern nachzuladen. Vorzugsweise wird dies nicht mit einzelnen Objektträgern durchgeführt, da die direkte Manipulation von Glas-Objektträgern (Glas-OTs) durch die üblichen Toleranzen der Objektträger-Abmessungen fehleranfällig ist. Statt dessen werden Objektträger in einen Rahmen (im Weiteren als Objektträgerrahmen bezeichnet) eingelegt, der dann als Ganzes gewechselt wird. Ein Objektträgerrahmen kann je nach Größe bzw. Gestaltung einen oder mehrere Objektträger aufnehmen.

Zur Aufnahme von Objektträgern in einem Objektträgerrahmen sind entsprechende Objektträgeraufnahmen vorgesehen, so dass in jede Objektträgeraufnahme jeweils ein Objektträger einsetzt werden kann.

Die Objektträgerrahmen werden beispielsweise in einem Magazin abgelegt, aus dem das Robotermodul die Objektträgerrahmen entnehmen kann. Nach einer Absuche des/der Objektträger(s), die in dem Objektträgerrahmen angeordnet sind, kann der jeweilige Objektträgerrahmen wieder in das Magazin zurückbefördert werden.

Weiterhin können Präparatewechsler vorgesehen sein, welche beispielsweise 1 bis 11 Magazine aufweisen, wobei jedes Magazin z.B. jeweils 16 Objektträgerrahmen à 5 Objektträger aufnehmen kann. Um den Objektträger bzw. mehrere Objektträger in einem Objektträgerrahmen aufnehmen zu können, weisen die Objektträgerrahmen für jeden Objektträger jeweils eine Objektträgeraufnahme auf.

Die Positioniergenauigkeit der Objektträgerrahmen selbst auf dem Scanningtisch ist über eine selbstzentrierende 3-Punkt-Auflage realisiert.

Die Objektträgerrahmen sind nicht exakt identisch, sondern weisen Fertigungstoleranzen auf. Zwar führen diese Fertigungstoleranzen in der Regel zu Positionierfehlern von wenigen Zehntelmillimetern, dies ist jedoch ausreichend, um beispielsweise eine Zelle bei der Relokalisation dieser Zelle basierend auf relativen Koordinaten aus dem Bildfeld des Mikroskops zu verschieben.

Entsprechend müssen die "absoluten" Koordinaten einer jeden Position auf einem Objektträger in Bezug auf einen Objektträgerrahmen, in welchen der Objektträger angeordnet ist, bekannt sein, um beliebige Koordinaten bezüglich eines Objekts, welches auf dem Objektträger aufgebracht ist, unabhängig vom verwendeten Objektträgerrahmen und der verwendeten Objektträgeraufnahme zuverlässig relokalisieren zu können.

In der Praxis kann es vorkommen, dass ein Objektträger nacheinander in verschiedene Objektträgerrahmen eingesetzt wird. Beispielsweise kann ein Objektträger eine Gewebeprobe enthalten, wobei diese Gewebeprobe eingefärbt wurde, um eine bestimmte Zelle(n) farblich zu markieren. Der Objektträger wird nun in einen "ersten" Objektträgerrahmen eingesetzt, um den Objektträger automatisch abzusuchen. Die eingefärbten Zellen werden hierbei erfasst und deren Position auf dem Objektträger festgehalten bzw. abgespeichert. Nach Abschluß dieser automatischen Absuche wird der Objektträger aus dem "ersten" Objektträgerrahmen entnommen, um eine Entfärbung des Objektträgers durchzuführen. Anschließend wird eine andere Färbung der Zellen durchgeführt, um zusätzliche Informationen über die zu untersuchenden Zellen erhalten zu können. Der Objektträger wird hierzu in einen "zweiten" Objektträgerrahmen eingesetzt und nochmals für eine Untersuchung unter ein Mikroskop gelegt. Um in einfacher Weise die zuvor untersuchten Zellen sofort weitergehend untersuchen zu können, ist es erforderlich, eine präzise Relokalisation durchführen zu können.

Für eine präzise Relokalisation muss daher die Position eines Objektträgers in jedem Objektträgerrahmen kalibriert werden, so dass ein Objektträger in verschiedene Objektträgerrahmen eingesetzt werden kann und die Positionierungskoordinaten eines "aufgefundenen" Objekts unabhängig von dem verwendeten Objektträgerrahmen zutreffen.

Gleiches gilt, wenn ein Objektträgerrahmen mehrere Objektträgeraufnahmen umfasst. In diesem Fall, muss für jede Objektträgeraufnahme eine Kalibrierung vorgenommen werden, sodass der gleiche Objektträger in jede Objektträgeraufnahme des Objektträgerrahmens eingesetzt werden könnte, und dennoch ein "aufgefundenes" Objekt zuverlässig relokalisiert werden kann.

Die Kalibrierung eines Objektträgerrahmen bzw. einer Objektträgeraufnahme wurde bisher derart durchgeführt, dass ein spezieller Objektträger (Kalibrierslide), der mit einem hochpräzisen Koordinatenraster versehen ist, in die zu kalibrierende Objektträgeraufnahme eingelegt wurde. Es wurden dann nacheinander drei definierte Koordinaten (drei Kalibrierpositionen) angefahren, welche mit einem Fadenkreuz zur Deckung zu bringen waren, das in einem Livebild auf einem Monitor des Mikroskop-Scanningsystems angezeigt wurde. Die zu diesen drei Kalibrierpositionen gehörigen Tischkoordinaten des Scanningtisches wurden von einer Software abgespeichert und dienten der Transformation von Koordinaten zwischen unterschiedlichen Positionen auf dem Objektträger bezüglich des Objektträgerrahmens.

Die aktuelle Generation von Präparatewechslern kann jedoch bis zu 880 Objektträger erfassen, so dass diese Vorgehensweise zum Kalibrieren von Objektträgerrahmen nicht mehr praktikabel ist.

Denn es ist hierbei erforderlich, für jede Objektträgeraufnahme eines Objektträgers in einem Objektträgerrahmen einen Einlegevorgang des Kalibrierslides vorzunehmen sowie drei präzise manuelle Positionieroperationen durchzuführen. Das interaktive Zentrieren der drei Rasterpositionen ist zudem zeitaufwendig. Sollen diese Schritte umgangen werden, ist eine hohe Anzahl (bspw. 80 Stück) identischer Kalibrierslides erforderlich, um ein Magazin mit Objektträgerrahmen automatisch kalibrieren zu können. Nachteilig bei diesem Vorgehen gemäß dem Stand der Technik sind die Kosten hochpräziser Kalibrierslides, wobei die Genauigkeit der Kalibrierung durch die Genauigkeit der Kalibrierslides limitiert ist. Die Herstellung von Kalibrierslides mit Toleranzen von 1 bis 5 µm ist aufwendig, und daher kostenintensiv.

US 5 694 212 A offenbart ein Verfahren und eine Vorrichtung zur Kalibrierung von Mikroskop-Objektträgern zur Verwendung in genauer und wiederholbarer Positionslokalisierung und Verlagerung bestimmter Bereiche einer Probe auf dem Objektträger, insbesondere bei Verwendung von Computer-korrelierten Positionen von Probenereignissen. Eine Abweichung von der Orthogonalität eines Objektträgers auf einem Mikroskoptisch (relativ zu einer Bewegung des Mikroskop-Objektträgertisches) wird bestimmt und mittels eines rechteckigen Kalibrierungsobjektträgers mit Positionsmarkierungen kompensiert.

US 5 428 690 A offenbart eine Vorrichtung und ein Verfahren zur automatischen Analyse von auf Objektträgern positionierten biologischen Proben. Die Vorrichtung umfasst ein interaktives optischen Subsystems zur Betrachtung der biologischen Probe auf dem Objektträger und zur Erzeugung eines interaktiven Videosignal, das dem betrachteten Bild entspricht. Ein automatisiertes optisches Subsystem beinhaltet ein einzelnes Hochleistungsmikroskopobjektiv zum Scannen eines Objektträgersatzes, Teile von dem bereits für die Analyse in den interaktiven optischen Mitteln identifiziert wurden. Ferner werden ein Verfahren und eine Vorrichtung zur Markierung von Stellen für eine spätere Analyse auf dem Mikroskopobjektträger und zum Assoziieren einer Analysefunktion mit jeder markierten Stelle beschrieben.

US 2003/027342 A1 offenbart ein Verfahren und eine Vorrichtung für eine erleichterte Erzeugung und Untersuchung von biologischen Substraten. Insbesondere wird die Positionierung von Materialien ermöglicht, die auf einem Substrat abgeschieden wurden, und mit großer Präzision übermittelt. Es ist ebenfalls möglich, auch kleine Bereiche von biologischen Materialien zu identifizieren, die auf einem Substrat abgeschieden sind, und wiederholt auf diese zuzugreifen, selbst wenn das biologische Substrat von dem Gerät entfernt wurde, das ursprünglich dazu verwendet wurde, um die Region von Interesse zu überprüfen, und dann wieder in das ursprüngliche Gerät eingesetzt wurde.

US 2005/051614 A1 offenbart eine Vorrichtung, ein System und ein Verfahren zum Analysieren von biologischen Proben, einschließlich Speichern der Analysedaten in einer am Objektträger befestigten Lese-/Schreib-Datenspeichervorrichtung. Die Datenspeichervorrichtung kann ein RFID-Tag, eine magnetische Vorrichtung oder eine optische Vorrichtung sein.

Erfindungsgemäß wird diese Aufgabe durch die Merkmale des unabhängigen Anspruchs gelöst. Bevorzugte Ausführungsformen der Erfindung sind Gegenstand der abhängigen Ansprüche.

Ein Aspekt der vorliegenden Erfindung betrifft ein Verfahren zur automatischen Kalibrierung von Objektträgern, insbesondere umfassend die Schritte:
- Bereitstellen eines Objektträgerrahmens mit mehreren Objektträgeraufnahmen, wobei Positionierungskoordinaten der Objektträgeraufnahmen bezüglich eines Mikroskop-Scanningsystems festgelegt sind;
- Bereitstellen zumindest eines Objektträgers mit einer Kalibrierungsmarkierung;
- Anordnen des zumindest einen Objektträgers in einer der Objektträgeraufnahmen des Objektträgerrahmens;
- Anordnen des Objektträgerrahmens mit dem Objektträger auf einem Scanningtisch des Mikroskop-Scanningsystems;
- Erfassen von Markierungskoordinaten der Kalibrierungsmarkierung basierend auf einer Detektion der Kalibrierungsmarkierung des Objektträgers durch das Mikroskop-Scanningsystem; und
- Erfassen einer Abweichung zwischen den Positionierungskoordinaten der Objektträgeraufnahme und den Markierungskoordinaten des Objektträgers.

Der Objektträgerrahmen ist insbesondere ein bereits kalibrierter Objektträgerrahmen. Umfasst ein kalibrierter Objektträgerrahmen mehrere Objektträgeraufnahmen, ist der Begriff "kalibrierter Objektträgerrahmen" derart zu verstehen, dass jede seiner Objektträgeraufnahmen kalibriert ist.

Umfasst ein Objektträgerrahmen mehrere bzw. eine Vielzahl von Objektträgeraufnahmen, so kann zumindest in eine (beliebige) Objektträgeraufnahme ein Objektträger eingesetzt bzw. angeordnet werden, um diesen Objektträger zu kalibrieren. Unter dem Begriff "Objektträgeraufnahme" ist insbesondere eine Position eines Objektträgers in bzw. auf einem Objektträgerrahmen zu verstehen. Auch umfasst der Begriff "Objektträgeraufnahme" einen mikroskopierbaren bzw. abscanbaren Bereich eines in einen Objektträgerrahmen einsetzbaren Objektträgers.

In anderen Worten ist ein kalibrierter Objektträgerrahmen bereits derart ausgemessen worden, dass die absoluten Positionierungskoordinaten eines Objektträgers, welcher in diesen Objektträgerrahmen bzw. eine Objektträgeraufnahme eingesetzt wird, bereits ausgemessen sind bzw. bekannt sind. Durch die absoluten Positionierungskoordinaten ist daher beispielsweise ein Koordinatenursprung, Mittelpunkt und/oder scanbarer Bereich des Objektträgers festgelegt, der über eine Eingabe von Scanningtisch-Koordinaten eines Mikroskop-Scanningsystems angefahren bzw. abgetastet werden kann.

Insbesondere sind die Positionierungskoordinaten der Objektträgeraufnahme, welche der kalibrierte Objektträgerrahmen umfasst, bekannt. Diese Positionierungskoordinaten können beispielsweise mit einem Koordinatenursprung und/oder einem Mittelpunkt eines eingelegten Objektträgers übereinstimmen. Dieser Mittelpunkt muss nicht der geometrische Mittelpunkt basierend auf den äußeren Abmaßen des Objektträgers sein, sondern kann auch ein Mittelpunkt sein, der einem Mittelpunkt des durch die Objektträgeraufnahme festgelegten scanbaren Bereich bzw. Scanningfenster entspricht.

Weiterhin können die Positionierungskoordinaten der Objektträgeraufnahme einen scanbaren Bereich bzw. Scanningfenster eines eingelegten Objektträgers bezüglich des Mikroskop-Scanningsystems festlegen.

Die Positionierungskoordinaten der Objektträgeraufnahme können insbesondere eine Ausrichtung bzw. Positionierung der Objektträgeraufnahme bzw. des in die Objektträgeraufnahme eingelegten Objektträgers bezüglich des Mikroskop-Scanningsystems festlegen. Die Positionierungskoordinaten der Objektträgeraufnahme können auch Positionierungskoordinaten des in die Objektträgeraufnahme eingelegten Objektträgers enthalten bzw. diesen entsprechen.

Beispielsweise kann ein Scanningtisch entsprechend der Positionierungskoordinaten der Objektträgeraufnahme eingestellt werden, um einen Objektträger abzusuchen und/oder ein bereits aufgefundenes Objekt zu relokalisieren.

Die Positionierungskoordinaten können insbesondere Koordinaten eines kartesischen Koordinatensystems entsprechen. Mit anderen Worten können die Positionierungskoordinaten X- und Y-Koordinaten zu einem oder mehreren Punkten bezüglich der Objektträgeraufnahme enthalten. Weiterhin können Positionierungskoordinaten auch Z-Koordinaten zu einem oder mehreren Punkten bezüglich der Objektträgeraufnahme enthalten. Die Positionierungskoordinaten können ggf. mittels Transformationsinformationen umgerechnet werden, so dass eine bestimmte Positionierungskoordinate(n) in eine Einstellung/Ausrichtung eines Scanningtischs umgerechnet wird/werden.

Die Positionierungskoordinaten sowie die Transformationsinformationen können beispielsweise in einem Speichermedium abgespeichert sein, welches von dem Mikroskop-Scanningsystem auslesbar ist.

Gemäß des Verfahrens kann in einen kalibrierten Objektträgerrahmen ein Objektträger mit einer Kalibrierungsmarkierung angeordnet bzw. eingelegt werden, wobei dieser Objektträger noch nicht kalibriert ist. Der kalibrierte Objektträgerrahmen mit dem (zu kalibrierenden) Objektträger wird auf einem Scanningtisch des Mikroskop-Scanningsystems angeordnet.

Die Kalibrierungsmarkierung kann zumindest einen Punkt, zumindest ein Kreuz oder zumindest eine Linie umfassen. Die Kalibrierungsmarkierung wird durch Abtasten bzw. Scannen von dem Mikroskop-Scanningsystem erfasst. Vorzugsweise wird die Kalibrierungsmarkierung automatisch von dem Mikroskop-Scanningsystem erkannt bzw. optisch erfasst. In anderen Worten wird die Kalibrierungsmarkierung des Objektträgers durch das Mikroskop-Scanningsystem detektiert. Beispielsweise kann hierzu eine Bilderkennung verwendet werden.

Basierend auf der Detektion der Kalibrierungsmarkierung erfasst das Mikroskop-Scanningsystem Markierungskoordinaten der Kalibrierungsmarkierung. Diese Markierungskoordinaten sind insbesondere X-Y-Koordinaten zu einem oder mehreren Punkten der Kalibrierungsmarkierung. Weiterhin sind diese Markierungskoordinaten auch Z-Koordinaten, welche eine Fokuslage definieren.

Die Markierungskoordinaten können relative Koordinaten basierend auf einem Koordinatenursprung des kalibrierten Objektträgerrahmens bzw. der Objektträgeraufnahme sein. Alternativ oder zusätzlich können die Markierungskoordinaten die Koordinaten des Scanningtisches bzw. der Messeinrichtung des Scanningtisches enthalten.

Zur Kalibrierung des Objektträgers mit der Kalibrierungsmarkierung wird die Abweichung der Markierungskoordinaten des Objektträgers von den Positionierungskoordinaten der kalibrierten Objektträgeraufnahme erfasst.

In anderen Worten ist die Abweichung eine Beabstandung der Markierungskoordinaten von den Positionierungskoordinaten.

Die Abweichung ist eine relative Größe hinsichtlich der Markierungskoordinaten im Bezug auf die Positionierungskoordinaten der (kalibrierten) Objektträgeraufnahme, wobei die Positionierungskoordinaten der (kalibrierten) Objektträgeraufnahme "absolute" Positionierungskoordinaten sind.

Die Abweichung kann auf einem Auswerten der Messdaten des Scanningstisches basieren. Beispielsweise kann ein Scanningstisch die Kalibrierungsmarkierung anfahren, vorzugsweise automatisch, und die Markierungskoordinaten als X₁ und Y₁ Koordinaten erfassen. Diese Markierungskoordinaten entsprechen den X₁ und Y₁ Koordinaten des Messsystems des Scanningtisches.

In einem anderen Schritt werden dann beispielsweise die Positionierungskoordinaten als X₂ und Y₂ Koordinaten der (kalibrierten) Objektträgeraufnahme ausgelesen, welche z.B. in einem Speichermedium abgespeichert wurden. Erforderlichenfalls können die ausgelesenen Positionierungskoordinaten im Hinblick auf eine Messskala des Scanningtisches transformiert werden.

Es wird eine Differenz Δx sowie Δy zwischen den Markierungskoordinaten X₁ und Y₁ und den Positionierungskoordinaten X₂ und Y₂ errechnet. Alternativ kann diese Differenz bestimmt werden, indem das Messsystem genullt wird, wenn die Markierungskoordinaten X₁ und Y₁ angefahren wurden.

Diese errechnete oder bestimmte Differenz wird als Abweichung abgespeichert bzw. festgehalten, so dass die Abweichung des Objektträgers mit der Kalibrierungsmarkierung bei Bedarf abgefragt werden kann.

In anderen Worten gibt die Abweichung einen Abstand in X-Richtung bzw. einen Abstand in Y-Richtung einer Markierungskoordinate der Kalibrierungsmarkierung von einer entsprechenden Positionierungskoordinate an, wenn die Abweichung in einem kartesischen Koordinatensystem erfasst wurde. Denkbar ist ebenfalls, dass andere Koordinatensysteme verwendet werden.

Somit kommt dem Erfassen einer Abweichung insbesondere die Bedeutung der Berechnung einer Differenz zwischen gemessenen Koordinaten der Markierungen und den Positionierungskoordinaten der Objektträgeraufnahme zu.

Insbesondere ist unter dem Begriff "Erfassen einer Abweichung" auch ein Zuordnen der zu einem bestimmten Objektträger erfassten Abweichung zu diesem bestimmten Objektträger zu verstehen. In anderen Worten ist die erfasste Abweichung für diesen einen Objektträger mit der Kalibrierungsmarkierung zutreffend.

Zusammengefasst ist ein Objektträger, der eine Kalibrierungsmarkierung aufweist und zu dem eine Abweichung erfasst wurde, ein kalibrierter Objektträger.

Vorzugsweise umfasst die Abweichung eine oder mehrere Abweichungskoordinate(n).

Insbesondere können die Abweichungskoordinaten des Objektträgers jeweils Abweichungen von Positionierungskoordinaten bezüglich verschiedener Punkte sein, welche im Hinblick auf eine Objektträgeraufnahme festgelegt wurden.

Beispielsweise können vier Eckpunkte der Objektträgeraufnahme als Positionierungskoordinaten festgelegt worden sein, wobei für jeden Eckpunkt jeweils X-Y-Koordinaten als Positionierungskoordinaten definiert wurden. In diesem Fall können Abweichungskoordinaten von den Positionierungskoordinaten zu jedem Eckpunkt erfasst bzw. festgelegt werden. In anderen Worten kann eine Abweichung mehrere Abweichungskoordinaten bezüglich verschiedener Punkte, die durch Positionierungskoordinaten definiert sind, enthalten.

Vorzugsweise können die Abweichungskoordinaten eine Abweichung der Markierungskoordinaten von Positionierungskoordinaten festlegen, wobei die Abweichungskoordinaten einen maximalen abtastbaren Scanbereich des Objektträgers festlegen. Beispielsweise kann ein Scanbereich durch mindestens drei Punkte festgelegt werden, wobei für jeden Punkt jeweilige Abweichungskoordinaten erfasst wurden.

Alternativ oder zusätzlich können die Abweichungskoordinaten eine Abweichung der Markierungskoordinaten von Positionierungskoordinaten festlegen, wobei die Abweichungskoordinaten einen Mittelpunkt des Objektträgers festlegen.

Weiterhin alternativ oder zusätzlich können die Abweichungskoordinaten eine Abweichung der Markierungskoordinaten von Positionierungskoordinaten festlegen, wobei die Abweichungskoordinaten zumindest zwei Referenzpunkte festlegen, die eine Transformation von Koordinaten der Objekträgeraufnahme im Bezug zu Koordinaten des Scanningtisches des Mikroskop-Scanningsystem festlegen.

Weiterhin vorzugsweise umfasst das Verfahren den Schritt:
- Hinterlegen der erfassten Abweichung in einem Speichermedium, welches mit dem Objektträger verbindbar ist.

Das Speichermedium ist vorzugsweise ein Barcode. Weiterhin kann das Speichermedium ein RFID-Etikett sein.

Eine Abweichung, welche zu einem bestimmten Objektträger erfasst wurde, ist im Regelfall nur für diesen Objektträger zutreffend. Entsprechend ist es erforderlich, die bezüglich eines bestimmten Objektträgers erfasste Abweichung diesem Objektträger zuverlässig zuzuordnen. Vorzugsweise kann eine zuverlässige Zuordnung des Objektträgers zu dessen Abweichung über eine "physische" Zuordnung des Objektträgers zu einem Speichermedium erfolgen, in welchem die erfasste Abweichung hinterlegt bzw. abgespeichert ist. Beispielsweise kann die erfasste Abweichung in einem Barcode hinterlegt werden, der als Speichermedium dient, wobei der Barcode mit dem Objektträger verbunden ist. Gleichermaßen kann ein RFID-Etikett (Radiofrequency Identification Etikett) anstelle oder zusätzlich zu einem Barcode verwendet werden.

Die hinterlegte bzw. erfasste Abweichung kann mittels einer Ausleseeinrichtung (bspw. Barcode- bzw. RFID-Auslesegerät) aus dem Speichermedium ausgelesen werden. Die Ausleseeinrichtung kann beispielsweise mit dem Mikroskop-Scanningsystem verbunden sein oder mit diesem verbindbar sein. Alternativ kann eine Ausleseeinrichtung beispielsweise mit einem Computer verbindbar sein und der Computer wiederum mit dem Mikroskop-Scanningsystem.

Weiterhin kann der Objektträger einen Identifikator, wie beispielsweise eine Nummer oder einen Barcode, umfassen. Die Abweichung kann zusammen mit einem Verweis auf den Identifikator oder zusammen mit dem Identifikator in einem Speichermedium hinterlegt sein, welches nicht physisch mit dem Objektträger verbunden ist. Zum Beispiel kann das Speichermedium in einem Computer angeordnet sein. Die Zuordnung von Abweichung zum jeweiligen Objektträger ist hierbei über den Identifikator sichergestellt.

Beispielsweise können über eine Schnittstelle am Mikroskop-Scanningsystem die erfassten Abweichungen dem Mikroskop-Scanningsystem zur Verfügung gestellt werden bzw. in dieses übertragen werden.

Vorzugsweise umfasst die Kalibrierungsmarkierung eine oder mehrere Linien und/oder Punkte.

Insbesondere können die Linien derart angeordnet sein, dass sich die Linien kreuzen. In anderen Worten ergeben die Linien bzw. Linierungen Kreuzungen bzw. Kreuzungspunkte. Einer oder mehrere solcher Kreuzungspunkte kann beispielsweise als Kalibrierungsmarkierung verwendet werden.

Weiterhin vorzugsweise ist die Kalibrierungsmarkierung auf einem transparenten Trägermaterial aufgebracht. Das transparente Trägermaterial kann lösbar mit dem Objektträger verbunden sein. Vorzugsweise ist das transparente Trägermaterial eine Klebefolie.

Vorteilhafterweise kann die Klebefolie schnell und einfach auf einen Objektträger, wie beispielsweise einen Glasobjektträger, aufgeklebt werden. Somit kann im Allgemeinen jeder beliebige Objektträger verwendet werden.

In einem anderen Ausführungsbeispiel ist die Kalibrierungsmarkierung direkt auf den Objektträger aufgebracht, wie beispielsweise aufgedruckt und/oder aufgelasert. Ebenfalls kann die Kalibrierungsmarkierung in dem Objektträger eingebracht bzw. eingebettet sein. Beispielsweise kann die Kalibrierungsmarkierung eine Faser bzw. ein Faden sein, die in den Objektträger eingebettet ist.

Weiterhin vorzugsweise wird die Kalibrierungsmarkierung durch Bedrucken des Objektträgers aufgebracht. Alternativ oder zusätzlich wird die Kalibrierungsmarkierung durch Bedrucken des transparenten Trägermaterials aufgebracht.

Weiterhin vorzugsweise umfasst der Objektträgerrahmen eine Vielzahl von Objektträgeraufnahmen, wobei in jede Objektträgeraufnahme jeweils ein Objektträger mit einer Kalibrierungsmarkierung eingesetzt wird bzw. eingesetzt werden kann. Somit kann für jeden Objektträger, der in dem Objektträgerrahmen angeordnet ist, eine Abweichung erfasst werden.

Vorteilhafterweise können so mehrere bzw. eine Vielzahl von Objektträger(n), die mit einer günstig hergestellten Kalibrierungsmarkierung versehen wurden, nacheinander automatisch kalibriert werden.

Das vorangehend beschriebene Verfahren zur automatischen Kalibrierung eines Objektträgers, ermöglicht vorteilhafterweise eine Kalibrierung von günstigen Objektträgern, wobei keine hohe Genauigkeit der Kalibrierungsmarkierung auf dem Objektträger erforderlich ist im Vergleich zu den aus dem Stand der Technik bekannten Kalibrierslides mit einem hochpräzisen Koordinatenraster.

Ein Aspekt der vorliegenden Erfindung betrifft ein Verfahren zur automatischen Kalibrierung von Objektträgerrahmen umfassend die Schritte:
- Bereitstellen zumindest eines Objektträgers mit einer Kalibrierungsmarkierung, wobei eine Abweichung von absoluten Positionierungskoordinaten des Objektträgers von der Kalibrierungsmarkierung des Objektträgers festgelegt ist;
- Anordnen des Objektträgers in einer Objektträgeraufnahme eines Objektträgerrahmens;
- Anordnen des Objektträgerrahmens mit dem Objektträger auf einem Scanningtisch eines Mikroskop-Scanningsystems;
- Erfassen von Markierungskoordinaten der Kalibrierungsmarkierung des Objektträgers basierend auf einer Detektion der Kalibrierungsmarkierung des Objektträgers durch das Mikroskop-Scanningsystem; und
- Festlegen von korrigierten Positionierungskoordinaten der Objektträgeraufnahme des Objektträgerrahmens basierend auf den Markierungskoordinaten und der Abweichung.

Der Objektträgerrahmen ist insbesondere ein nicht-kalibrierter oder ein unzureichend kalibrierter Objektträgerrahmen. In anderen Worten können Objekte auf einem Objektträger, die in einen nicht-kalibrierten oder nur unzureichend kalibrierten Objektträgerrahmen eingesetzt werden, nur schlecht bzw. unzureichend relokalisiert werden. Entsprechend ist eine Überprüfbarkeit von Objektträgern, die in einen solchen unzureichend kalibrierten Objektträgerrahmen eingesetzt werden, nur bedingt oder nicht durch ein automatisches Scannen bzw. Abtasten möglich.

Umfasst ein nicht-kalibrierter Objektträgerrahmen mehrere Objektträgeraufnahmen, ist der Begriff "nicht-kalibrierter Objektträgerrahmen" derart zu verstehen, dass zumindest eine oder sogar jede dieser Objektträgeraufnahmen nicht-kalibriert ist/sind.

Unter dem Begriff "Objektträgeraufnahme" ist insbesondere eine Position eines Objektträger in bzw. auf einem Objektträgerrahmen zu verstehen. Auch umfasst der Begriff "Objektträgeraufnahme" einen mikroskopierbaren bzw. abscanbaren bzw. abtastbaren Bereich eines in einen Objektträgerrahmen einsetzbaren Objektträgers.

In anderen Worten ist bei einem nicht-kalibrierten Objektträgerrahmen nicht sichergestellt, dass die absoluten Positionierungskoordinaten eines Objektträgers, welcher in diesen Objektträgerrahmen eingesetzt wird, zutreffen bzw. mit den tatsächlichen Positionierungskoordinaten des Objektträgerrahmens auf dem Scanningtisch übereinstimmen. Denn es ist möglicherweise eine Korrektur bzw. Anpassung der absoluten Positionierungskoordinaten des Objektträgers auf die Positionierungskoordinaten des (nicht-kalibrierten) Objektträgerrahmens erforderlich.

Ein zutreffender Koordinatenursprung, Mittelpunkt und/oder scanbarer Bereich des Objektträgers im Bezug auf den Objektträgerrahmen ist daher nicht sichergestellt.

Vielmehr kann es zu einer Verschiebung des Objektträgers bezüglich des mikroskopierbaren bzw. scanbaren Bereichs kommen, weil eine nicht bekannte Differenz zwischen den bekannten absoluten Positionierungskoordinaten des Objektträgers und den tatsächlich erforderlichen Koordinaten, die auf dem Scanningtisch bezüglich des Objektträgerrahmens eingestellt müssten, existiert.

Eine Eingabe von Scanningtisch-Koordinaten hinsichtlich eines Objektes auf einem Objektträger, der in diesem nicht-kalibrierten Objektträgerrahmen eingelegt ist, kann möglicherweise zu keinen oder einem falschen Objekt führen.

Weiterhin gibt es keinen mechanischen Ursprung, welcher ein "absolutes" Koordinatensystem festlegt. Vielmehr müssen "absolute Koordinaten" bzw. "absolute Positionierungskoordinaten" praktisch nach (jeder) Montage eines (Scanning-)Tisches an das Mikroskop festgelegt werden, indem ein präzises Kalibrierslide (z.B. Micropositioner der Firma Berliner Glas) in eine Objektträgeraufnahme eines Objektträgerrahmens eingelegt wird und 3 vordefinierte Markierungen des Kalibrierslides unter dem Mikroskopobjektiv nacheinander zentriert werden (mit visueller Kontrolle durch Okulare). Die zugehörigen Tischkoordinaten werden gespeichert und zur Bestimmung eines absoluten Koordinatensystems verwendet.

Basierend auf einem solchen (festgelegten) "absoluten Koordinatensystem", können Objektträgeraufnahmen von Objektträgerrahmen mittels des Verfahrens zur automatischen Kalibrierung von Objektträgerrahmen kalibriert werden.

In anderen Worten werden zu kalibrierende Objektträger bzw. zu kalibrierende Objektträgerrahmen relativ zu diesem absoluten Koordinatensystem kalibriert.

Insbesondere sind unter der Begrifflichkeit "absolute Koordinaten" bzw. "absoluten Positionierungskoordinaten" festgelegte Positionierungskoordinaten eines kalibrierten Objektträgers zu verstehen, welche eine bestimmte Position im absoluten Koordinatensystem festlegen bzw. bestimmen. Insbesondere dienen absolute Positionierungskoordinaten dazu, einen Objektträger basierend auf diesen absoluten Positionierungskoordinaten "auszurichten" bzw. "zu zentrieren".

Die absoluten Positionierungskoordinaten eines kalibrierten Objektträgers sind bekannt bzw. wurden bereits festgelegt. Diese absoluten Positionierungskoordinaten können beispielsweise einen Koordinatenursprung und/oder einen Mittelpunkt des Objektträgers festlegen bzw. definieren. Weiterhin können die absoluten Positionierungskoordinaten des Objektträgers einen scanbaren Bereich bzw. Scanningfenster des Objektträgers bezüglich des Mikroskop-Scanningsystems festlegen. Die absoluten Positionierungskoordinaten des Objektträgers können insbesondere eine Ausrichtung bzw. Positionierung des Objektträgers bezüglich des Mikroskop-Scanningsystems festlegen. Die absoluten Positionierungskoordinaten der Objektträgeraufnahme können Positionierungskoordinaten einer kalibrierten Objektträgeraufnahme bzw. eines Objektträgerrahmens enthalten bzw. diesen entsprechen.

Insbesondere sind die absoluten Positionierungskoordinaten die Positionierungskoordinaten, die für einen bestimmten kalibrierten Objektträger festgelegt wurden, als dieser Objektträger in der kalibrierten Objektträgeraufnahme angeordnet war, um die Positionierungskoordinaten festzulegen/zu erfassen. Vorzugsweise wurden die Positionierungskoordinaten des nun kalibrierten Objektträgers mittels des Verfahrens zur automatischen Kalibrierung von Objektträgern erfasst bzw. festgelegt.

Die absoluten Positionierungskoordinaten können insbesondere Koordinaten eines kartesischen Koordinatensystems entsprechen. Mit anderen Worten können die absoluten Positionierungskoordinaten X- und Y-Koordinaten zu einem oder mehreren Punkten bezüglich des Objektträgers enthalten.

Zusätzlich können die absoluten Positionierungskoordinaten auch Z-Koordinaten enthalten.

Die absoluten Positionierungskoordinaten können ggf. mittels Transformationsinformationen umgerechnet werden, so dass eine bestimmte Positionierungskoordinate(n) in eine Einstellung/Ausrichtung bzw. Messskala eines Scanningtischs umgerechnet wird/werden. Die absoluten Positionierungskoordinaten sowie die Transformationsinformationen können beispielsweise in einem Speichermedium abgespeichert sein, welches von dem Mikroskop-Scanningsystem auslesbar ist.

Gemäß dem Verfahren wird in einen nicht-kalibrierten Objektträgerrahmen ein Objektträger mit einer Kalibrierungsmarkierung angeordnet bzw. eingelegt, wobei der Objektträger mit einer Kalibrierungsmarkierung kalibriert ist. Der nicht-kalibrierte Objektträgerrahmen mit dem kalibrierten Objektträger wird auf einem Scanningtisch des Mikroskop-Scanningsystems angeordnet.

Die Kalibrierungsmarkierung kann zumindest ein Punkt, zumindest ein Kreuz oder zumindest eine Linie umfassen. Die Kalibrierungsmarkierung wird durch Abtasten bzw. Scannen von dem Mikroskop-Scanningsystem erfasst. Vorzugsweise wird die Kalibrierungsmarkierung automatisch von dem Mikroskop-Scanningsystem erkannt bzw. optisch erfasst. In anderen Worten wird die Kalibrierungsmarkierung des Objektträgers durch das Mikroskop-Scanningsystem detektiert.

Basierend auf der Detektion der Kalibrierungsmarkierung erfasst das Mikroskop-Scanningsystem Markierungskoordinaten der Kalibrierungsmarkierung. Diese Markierungskoordinaten sind insbesondere X-Y-Koordinaten zu einem oder mehreren Punkten der Kalibrierungsmarkierung. Anstatt kartesischer Koordinaten können auch andere Koordinatensysteme verwandt werden. Insbesondere können Markierungskoordinaten zusätzlich zu den X-Y-Koordinaten auch Z-Koordinaten enthalten.

Die Markierungskoordinaten können die Koordinaten des Scanningtisches bzw. der Messeinrichtung des Scanningtisches enthalten.

Zur Kalibrierung des Objektträgerrahmens werden korrigierte Positionierungskoordinaten festgelegt, die ausgehend von den Markierungskoordinaten des Objektträgers und der Abweichung bestimmt werden.

In anderen Worten sollen die absoluten Positionierungskoordinaten des Objektträgers mit den korrigierten Positionierungskoordinaten für den Objektträgerrahmen bzw. der Objektträgeraufnahme übereinstimmen. Um eine Übereinstimmung erzielen zu können, ist es erforderlich, zuerst die Kalibrierungsmarkierung anzufahren. Hierdurch können die X-Y-Koordinaten des Scanningtisch im Hinblick auf die Kalibrierungsmarkierung in Erfahrung gebracht werden. Diese X-Y-Koordinaten des Scanningtisch werden als Markierungskoordinaten erfasst. Somit ist die Positionierung der Kalibrierungsmarkierung in dem Objektträgerrahmen bekannt bzw. festgelegt.

In einem nächsten Schritt kann dann die korrekte Positionierung des Objektträgerrahmens bzw. die korrekte Einstellung des Scanningtisches bestimmt werden. Hierzu wird eine korrigierte Positionierung des Objektträgerrahmens basierend auf einer Berücksichtigung der bereits bekannten Beabstandung bzw. Abweichung der absoluten Positionierungskoordinaten (bspw. Mittelpunkt des Objektträgers, etc.) von der Kalibrierungsmarkierung/ Markierungskoordinaten festgestellt. Beispielsweise kann die Abweichung ausgehend von den Markierungskoordinaten mit dem Scanningtisch angefahren bzw. eingestellt, so dass dann die Einstellungen (X- und Y-Koordinate, ggf. Z-Koordinate bezüglich einer Fokuslage) des Scanningtisches bekannt sind, die tatsächlich mit den Punkten, die durch die absoluten Positionierungskoordinaten des Objektträgers definiert werden, übereinstimmen bzw. kongruent sind.

Alternativ oder zusätzlich können die korrigierten Positionierungskoordinaten basierend auf den Markierungskoordinaten unter Berücksichtigung der Abweichung ausgerechnet werden.

Die Abweichung ist eine relative Größe hinsichtlich der Markierungskoordinaten im Bezug auf die absoluten Positionierungskoordinaten des kalibrierten Objektträgers. Die Punkte, die durch die korrigierten Positionierungskoordinaten der kalibrierten Objektträgeraufnahme festgelegt sind, sind deckungsgleich mit den Punkten, die durch die absoluten Positionierungskoordinaten des Objektträgers festgelegt sind.

Vorzugsweise entspricht die (festgelegte) Abweichung/ der Abstand zwischen den absoluten Positionierungskoordinaten des Objektträgers und der Kalibrierungsmarkierung des Objektträgers der erfassten Abweichung zwischen den Markierungskoordinaten der Kalibrierungsmarkierung des Objektträgers und den Positionierungskoordinaten einer (kalibrierten) Objektträgeraufnahme, welche im Rahmen des Verfahrens zur automatischen Kalibrierung von Objektträgern erfasst bzw. bestimmt wurde. Folglich wird die (festgelegte) Abweichung beim Kalibrieren der Objektträgeraufnahme verwendet, um basierend auf den Markierungskoordinaten der Kalibrierungsmarkierung die genauen, nämlich korrigierten Positionierungskoordinaten festzulegen bzw. zu berechnen.

Die entsprechenden X- und Y-Koordinaten des Messsystems des Scanningtisches hinsichtlich der korrigierten Positionierungskoordinaten werden für Objektträgerrahmen bzw. Objektträgeraufnahme erfasst. Objektträgerrahmen bzw. Objektträgeraufnahme sind somit kalibriert.

Die vorherigen Ausführungen bezüglich der Abweichung sind bezüglich dieses Verfahrens zur Kalibrierung von Objektträgerrahmen ebenfalls zutreffend.

Insbesondere ist unter dem Begriff "Festlegen von korrigierten Positionierungskoordinaten" auch ein Zuordnen der zu einem bestimmten Objektträgerrahmen bzw. -aufnahme festgelegten korrigierten Positionierungskoordinaten zu diesem bestimmten Objektträgerrahmen bzw. -aufnahme zu verstehen. In anderen Worten sind die festgelegten korrigierten Positionierungskoordinaten nur für diese(n) eine(n) Objektträgerrahmen bzw. -aufnahme zutreffend.

Zusammengefasst ist ein(e) Objektträgerrahmen bzw. -aufnahme, für den/die korrigierte Positionierungskoordinaten festgelegt bzw. festgestellt wurden, ein(e) kalibrierte(r) Objektträgerrahmen bzw. -aufnahme.

In einem anderen Schritt werden dann beispielsweise die korrigierten Positionierungskoordinaten der (kalibrierten) Objektträgeraufnahme ausgelesen, welche z.B. in einem Speichermedium abgespeichert wurden, um einen anderen Objektträger abzutasten oder Objekte auf diesem Objektträger zu relokalisieren.

Vorzugsweise legen die korrigierten Positionierungskoordinaten der Objektträgeraufnahme einen maximalen abtastbaren Scanbereich eines in der Objekträgeraufnahme anordenbaren Objektträgers fest.

Insbesondere können die korrigierten Positionierungskoordinaten der Objektträgeraufnahme jeweils Positionierungskoordinaten zu verschiedenen Punkten sein, welche im Hinblick auf einen einlegbaren Objektträger festgelegt wurden. Beispielsweise können vier Eckpunkte eines Objektträgers als absolute Positionierungskoordinaten festgelegt worden sein, wobei für jeden Eckpunkt jeweils X-Y-Koordinaten als absolute Positionierungskoordinaten definiert wurden. In diesem Fall können korrigierte Positionierungskoordinaten hinsichtlich der absoluten Positionierungskoordinaten eines jeden Eckpunkts ausgehend von den Markierungskoordinaten erfasst bzw. festgelegt werden. In anderen Worten können korrigierte Positionierungskoordinaten mehrere Positionierungskoordinaten bezüglich verschiedener Punkte enthalten, die durch voneinander verschiedene, korrigierte Positionierungskoordinaten definiert sind.

Alternativ oder zusätzlich können die korrigierten Positionierungskoordinaten der Objektträgeraufnahme einen Mittelpunkt eines in der Objekträgeraufnahme anordenbaren Objektträgers festlegen. Beispielsweise kann ein Scanbereich durch mindestens drei Punkte festgelegt werden, wobei für jeden Punkt jeweilige korrigierte Positionierungskoordinaten erfasst wurden.

Weiterhin alternativ oder zusätzlich können die korrigierten Positionierungskoordinaten der Objektträgeraufnahme zumindest zwei Referenzpunkte festlegen, die eine Transformation von Koordinaten eines in der Objekträgeraufnahme anordenbaren Objekträgers in Bezug auf Koordinaten des Scanningtisches des Mikroskop-Scanningsystem festlegen.

Vorzugsweise umfasst das Verfahren weiterhin den Schritt:
- Hinterlegen der korrigierten Positionierungskoordinaten auf einem Speichermedium, welches vorzugsweise mit dem Objektträgerrahmen verbunden ist, wobei das Speichermedium vorzugsweise ein Barcode oder ein RFID-Etikett ist.

Das Speichermedium ist vorzugsweise ein Barcode. Weiterhin kann das Speichermedium ein RFID-Etikett sein.

Korrigierte Positionierungskoordinaten, welche zu einem/einer bestimmten Objektträgerrahmen bzw. -aufnahme erfasst wurden, sind im Regelfall nur für diese(n) Objektträgerrahmen bzw. -aufnahme zutreffend. Entsprechend ist es erforderlich, die bezüglich eines/einer bestimmten Objektträgerrahmens bzw. -aufnahme festgelegten korrigierten Positionierungskoordinaten diesem/dieser Objektträgerrahmen bzw. - aufnahme zuverlässig zuzuordnen.

Vorzugsweise kann eine zuverlässige Zuordnung von Objektträgerrahmen bzw. - aufnahme zu dessen korrigierten Positionierungskoordinaten über eine "physische" Zuordnung von Objektträgerrahmen bzw. -aufnahme zu einem Speichermedium erfolgen, in welchem die korrigierten Positionierungskoordinaten hinterlegt bzw. abgespeichert sind. Beispielsweise können die korrigierten Positionierungskoordinaten in einem Barcode hinterlegt werden, der als Speichermedium dient, wobei der Barcode mit Objektträgerrahmen bzw. -aufnahme verbunden ist. Gleichermaßen kann ein RFID-Etikett (Radiofrequency Identification Etikett) anstelle oder zusätzlich zu einem Barcode verwendet werden.

Die hinterlegten bzw. festgelegten korrigierten Positionierungskoordinaten können mittels einer Ausleseeinrichtung (bspw. Barcode- bzw. RFID-Auslesegerät) aus dem Speichermedium ausgelesen werden. Die Ausleseeinrichtung kann beispielsweise mit dem Mikroskop-Scanningsystem verbunden sein oder mit diesem verbindbar sein. Alternativ kann eine Ausleseeinrichtung beispielsweise mit einem Computer verbindbar sein und der Computer wiederum mit dem Mikroskop-Scanningsystem.

Weiterhin kann der/die Objektträgerrahmen bzw. -aufnahme einen Identifikator, wie beispielsweise eine Nummer oder einen Strichcode bzw. Barcode, umfassen.

Insbesondere kann ein Strichcode eine 1D-Codierung (eindimensionale Codierung) und/oder 2D-Codierung (zweidimensionale Codierung) aufweisen. Beispielsweise kann ein 1D-Code wie ein üblicher Handelsstrichcode gestaltet sein. Ein üblicherweise verwendeter bzw. gängiger Handelsstrichcode ist beispielsweise ein EAN (European Article Number) oder ein UPC (Universal Product Code).

Die korrigierten Positionierungskoordinaten können zusammen mit einem Verweis auf den Identifikator oder zusammen mit dem Identifikator in einem Speichermedium hinterlegt sein, welches nicht physisch mit dem Objektträgerrahmen verbunden ist. Zum Beispiel kann das Speichermedium in einem Computer angeordnet sein. Die Zuordnung von korrigierten Positionierungskoordinaten zum jeweiligen Objektträgerrahmen ist über den Identifikator sichergestellt. Weist ein Objektträgerrahmen mehrere Objektträgeraufnahmen auf, kann jeder Objektträgeraufnahme über ein entsprechenden Identifikator oder direkt die korrigierten Positionierungskoordinaten zugeordnet werden.

Beispielsweise können über eine Schnittstelle am Mikroskop-Scanningsystem die erfassten korrigierten Positionierungskoordinaten dem Mikroskop-Scanningsystem zur Verfügung gestellt werden bzw. in dieses übertragen werden.

Vorzugsweise umfasst die Kalibrierungsmarkierung Linierungen und/oder Punkte.

Insbesondere können die Linien derart angeordnet sein, dass sich die Linien kreuzen. In anderen Worten ergeben die Linien bzw. Linierungen Kreuzungen bzw. Kreuzungspunkte. Einer oder mehrere solcher Kreuzungspunkte kann beispielsweise als Kalibrierungsmarkierung verwendet werden.

Weiterhin vorzugsweise ist die Abweichung ebenfalls in einem Speichermedium hinterlegt, und die Abweichung aus diesem Speichermedium auslesbar, so dass in einfacher Weise korrigierte Positionierungskoordinaten festgelegt werden können. Vorteilhafterweise ist die Abweichung maschinell auslesbar, so dass automatisch korrigierte Positionierungskoordinaten errechnet bzw. bestimmt werden können.

Weiterhin vorzugsweise umfasst das Verfahren den Schritt:
- Auslesen eines Speichermediums, welches mit dem Objektträger verbunden ist, und in welchem die Abweichung hinterlegt ist.

Vorteilhafterweise ist in diesem Fall die Abweichung sofort verfügbar, wenn der Objektträger verwendet wird, weil das Speichermedium und der Objektträger miteinander verbunden sind.

Hierbei wird vorteilhafterweise auch vermieden, dass eine fehlerhafte Zuordnung einer Abweichung zu einem anderen und somit falschen Objektträger geschieht, was zu inkorrekten bzw. falschen korrigierten Positionierungskoordinaten führen würde.

Vorzugsweise umfasst der Objektträgerrahmen eine Vielzahl von Objektträgeraufnahmen, wobei in jede Objektträgeraufnahme ein Objektträger mit einer Kalibrierungsmarkierung eingesetzt wird, um für jede Objektträgeraufnahme korrigierte Positionierungskoordinaten festzulegen.

Vorteilhafterweise können so mehrere bzw. eine Vielzahl von Objektträgeraufnahmen nacheinander automatisch kalibriert werden.

Das vorangehend beschriebene Verfahren zur automatischen Kalibrierung eines Objektträgerrahmens, ermöglicht vorteilhafterweise eine Kalibrierung von Objektträgerrahmen basierend auf kostengünstigen und einfach herzustellenden Objektträgern.

Vorzugsweise können einer oder mehrere Verfahrensschritte des Verfahrens zur automatischen Kalibrierung eines Objektträgers in Kombination mit einen oder mehreren Verfahrensschritten des Verfahrens zur automatischen Kalibrierung eines Objektträgerrahmens verwendet werden. Insbesondere können die beiden Verfahren in Kombination durchgeführt werden.

Ein Aspekt der vorliegenden Erfindung betrifft einen Objektträger umfassend
- ein Objektträgersubstrat und
- eine Kalibrierungsmarkierung,
wobei die Kalibrierungsmarkierung mit dem Objektträgersubstrat verbunden ist, und wobei der Objektträger geeignet ist eine Kalibrierung gemäß einem vorangehend beschriebenen Verfahren zur Kalibrierung von Objektträgerrahmen durchzuführen.

Vorzugsweise ist ein Objektträgersubstrat eine Glas- oder Kunststoffplatte, welche üblicherweise als Objektträger in der Mikroskopie verwendet wird.

Die Kalibrierungsmarkierung umfasst vorzugsweise eine oder mehrere Linien und/oder Punkte. Weiterhin vorzugsweise ist die Kalibrierungsmarkierung auf ein transparentes Trägermaterial aufgebracht. In einer weiter bevorzugten Ausführungsform ist die Kalibrierungsmarkierung auf das transparente Trägermaterial aufgedruckt.

Vorzugsweise umfasst die Kalibrierungsmarkierung mindestens zwei automatisch erkennbare Merkmale, die mindestens zwei (Referenz-) Koordinaten festlegen bzw. erlauben solche (Referenz-) Koordinaten festzulegen.

Vorzugsweise ist das transparente Trägermaterial eine Folie. Das transparente Trägermaterial ist bevorzugt stoffschlüssig mit dem Objektträgersubstrat verbunden. Das transparente Trägermaterial kann als Klebefolie ausgestaltet sein, dass auf das Objektträgersubstrat aufgeklebt wird.

In anderen Worten kann ein Objektträger-Glasplättchen mit einer durchsichtigen Klebefolie beklebt werden.

Zusammengefasst beruht die vollautomatische Kalibrierung von Objektträgerrahmen auf einfach und kostengünstig herzustellenden Kalibrierslides, die automatisch erkennbare Markierungen enthalten.

Vorteilhafterweise entfällt somit das interaktive Zentrieren der drei Rasterpositionen, wie es im Stand der Technik üblich ist. Vorteilhafterweise wird auch der zweite interaktive Schritt des Einlegens des Kalibrierslides in die jeweils nächste Objektträgeraufnahme gemäß dem Stand der Technik überflüssig.

Auch wird es vorteilhafterweise überflüssig eine hohe Anzahl (bspw. 80 Stück) identischer Kalibrierslides bereitzustellen, um ein Magazin mit Objektträgerrahmen gemäß dem Stand der Technik automatisch kalibrieren zu können. Nachteilig bei diesem Vorgehen gemäß dem Stand der Technik sind die Kosten hochpräziser Kalibrierslides, wobei die Genauigkeit der Kalibrierung durch die Genauigkeit der Kalibrierslides limitiert ist. Die Herstellung von Kalibrierslides mit Toleranzen von 1 bis 5 µm ist inakzeptabel teuer.

Insbesondere sind die vorliegenden Verfahren vorteilhaft, wenn viele Objektträgerrahmen sowie Objektträgeraufnahmen kalibriert werden sollen. In diesem Fall ist insbesondere eine hohe Zeitersparnis im Vergleich zu Verfahren im Stand der Technik möglich.

Zudem sind die vorliegenden Verfahren auch dann vorteilhaft, wenn ein automatisches Scanningsystem mehr als eine einzige Position für einen Objektträger aufweist. In anderen Worten sind die erfindungsgemäßen Verfahren sowie der erfindungsgemäße Objektträger vorteilhaft, sobald Objektträgerrahmen mit mehreren Objektträgeraufnahmen und/oder eine Vielzahl Objektträgerrahmen verwendet werden und sichergestellt sein soll, dass eine genaue Relokalisierung von Objekten möglich ist, wenn die verwendeten Objektträger nicht immer in dieselben Objekträgeraufnahmen eingelegt werden. In anderen Worten sind die erfindungsgemäßen Verfahren und der Objektträger vorteilhaft, sobald eine zuverlässige Relokalisierung bezüglich eines Objekts auf einem Objektträger in verschiedenen Objekträgeraufnahmen sichergestellt werden soll.

Die Ausführungen, Beschreibungen und Definition bezüglich der voranstehend beschriebenen Verfahren treffen ebenfalls auf den erfindungsgemäßen Objektträger hinsichtlich seiner Ausgestaltung und seiner Verwendung zu.

Die Erfindung wird nachstehend anhand von Ausführungsbeispielen unter Bezugnahme auf die beigefügten Zeichnungen beispielhaft beschrieben. Insbesondere können einzelne Merkmale der vorangehend und/oder nachfolgend beschrieben Aspekte und/oder Ausführungsformen losgelöst von dem jeweiligen Aspekt bzw. der jeweiligen Ausführungsform zu weiteren Ausführungsformen kombiniert werden.

Es zeigt:
Fig. 1: ein Mikroskop-Scanningsystem;
Fig. 2: schematisch dargestellt einen Scanningtisch mit einem Objektträgerrahmen und acht eingelegten Objektträgern; und
Fig. 3: einen Objektträger mit einer Kalibrierungsmarkierung.

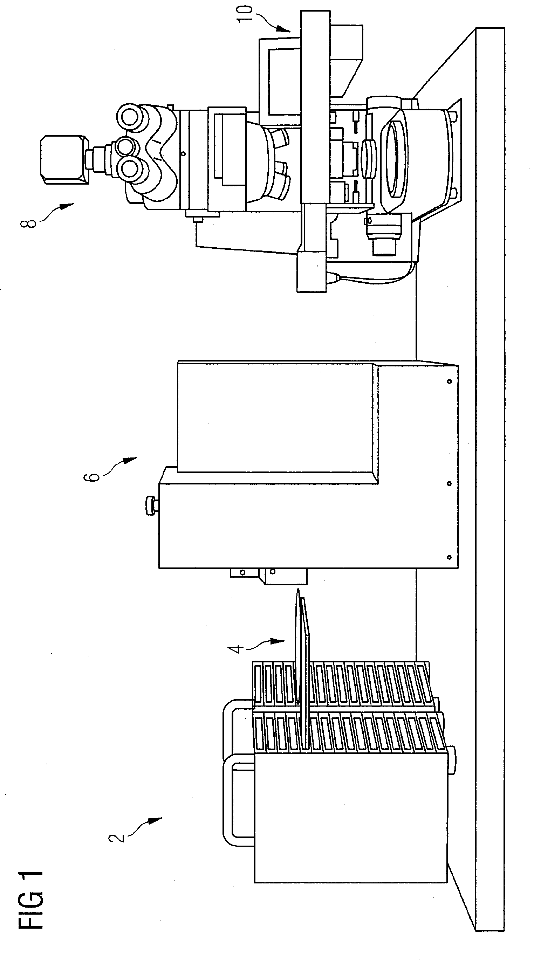

**Figur 1** zeigt ein Mikroskop-Scanningsystem 100, welches ein Magazin 2, ein Robotermodul 6 und ein Scanning-Mikroskop 8 mit einem Scanningtisch 10 umfasst.

Das Magazin 2 umfasst eine Vielzahl von Objektträgerrahmen 4, beispielsweise 16 Objektträgerrahmen 4. Mittels des Robotermoduls 6 kann ein Objektträgerrahmen 4 aus dem Magazin 2 entnommen werden und auf dem Scanning-Mikroskop 8 angeordnet bzw. positioniert werden.

Es kann beispielsweise jeweils ein Objektträgerrahmen 4 auf dem Scanning-Mikroskop 8 angeordnet bzw. positioniert werden. Hierzu entnimmt das Robotermodul 6 einen Objektträgerrahmen 4. Jeder Objektträgerrahmen 4 kann beispielsweise fünf Objektträgeraufnahmen umfassen, in welche jeweils ein Objektträger einlegbar bzw. einsetzbar bzw. anordenbar ist. Die Objektträger sind insbesondere ortsfest in die Objektträgerrahmen einsetzbar bzw. in diesen ausrichtbar. Beispielsweise könnten Objektträger mittels magnetischer Befestigung, oder formschlüssigen Anordnungs- bzw. Ausrichtungseinrichtungen auf/in den Objektträgerrahmen angeordnet werden. In anderen Worten kann mittels einer geeigneten Befestigung sichergestellt werden, dass Objektträger immer gleich auf einem Objektträgerrahmen positioniert bzw. angeordnet werden, so dass eine gute Reproduzierbarkeit bezüglich einer Positionierung von Objektträgern auf dem Objektträgerrahmen gewährleistet ist.

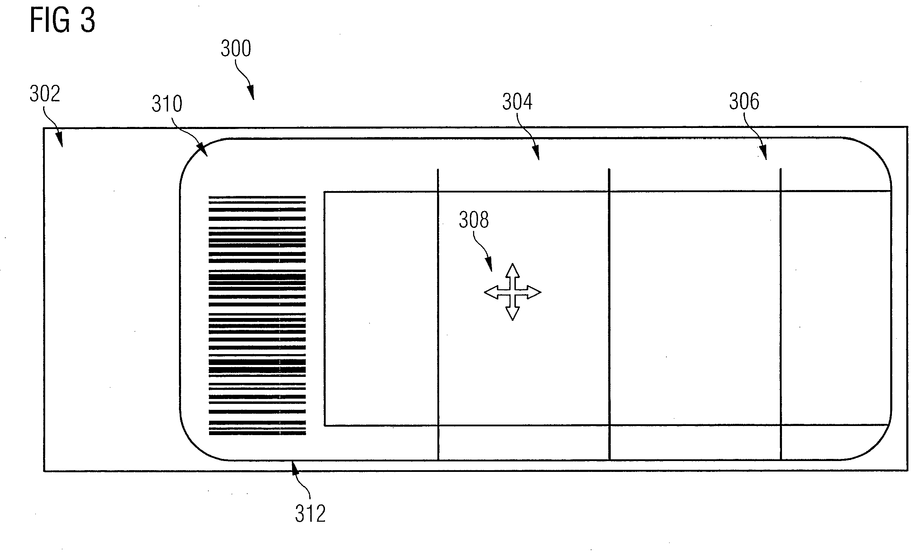

Zur automatischen Kalibrierung von Objektträgern kann beispielsweise ein kalibrierter Objektträgerrahmen 4 mit einer Objektträgeraufnahme aus dem Magazin 2 von dem Robotermodul 6 entnommen werden und auf den Scanningtisch 10 des Scanning-Mikroskops 8 angeordnet werden. Der Objektträgerrahmen 4 ist kalibriert, so dass Positionierungskoordinaten der Objektträgeraufnahme bezüglich eines Mikroskop-Scanningsystems festgelegt sind. In der Objektträgeraufnahme ist beispielsweise ein nicht kalibrierter Objektträger angeordnet, der mit einer Kalibrierungsmarkierung versehen ist. Ein solcher Objektträger ist beispielsweise vergleichbar mit dem Objektträger 300, wie er in **Figur 3** dargestellt ist und nachstehend beschrieben wird.

Zum Kalibrieren dieses Objektträgers 300 mit einer Kalibrierungsmarkierung 304 wird die Kalibrierungsmarkierung 304 des Objektträgers 300 durch das Mikroskop-Scanningsystem 100, insbesondere durch das Scanning-Mikroskop 8 detektiert. Hierbei erkennt bzw. erfasst das Scanning-Mikroskop 8 die Kalibrierungsmarkierung 304 und erfasst gleichzeitig Markierungskoordinaten dieser Kalibrierungsmarkierung 304. Diese Markierungskoordinaten können Messkoordinaten des Scanningtisches 10 sein.

Die Kalibrierungsmarkierung 304 bzw. deren Markierungskoordinaten stimmen möglicherweise nicht mit den Positionierungskoordinaten der kalibrierten Objektträgeraufnahme überein. In anderen Worten sind der/die Punkt(e), die die Markierungskoordinaten festlegen bzw. definieren, nicht deckungsgleich bzw. identisch mit dem/den Punkt(en), die die Positionierungskoordinaten festlegen bzw. definieren. In diesem Fall sind die (Koordinaten-) Einstellungen des Scanningtisches 10 für die Markierungskoordinaten andere als für die Positionierungskoordinaten. Somit besteht eine Differenz bzw. eine Abweichung zwischen den Positionierungskoordinaten der Objektträgeraufnahme und den Markierungskoordinaten des Objektträgers 300.

Zum Kalibrieren des Objektträgers 300 wird diese Abweichung 308 bzw. der Abstand der Kalibrierungsmarkierung 304 des Objektträgers 300 von den Positionierungskoordinaten der Objektträgeraufnahme erfasst bzw. bestimmt.

Wurden durch die Positionierungskoordinaten der Objektträgeraufnahme beispielsweise ein Mittelpunkt festgelegt, so ergeben sich beispielsweise durch ein Addieren der Markierungskoordinaten und der Abweichung Mittelpunktskoordinaten des Objektträgers 300.

Bei einem Verfahren zur automatischen Kalibrierung von Objektträgerrahmen wird ein nach dem obigen Verfahren beschriebener, kalibrierter Objektträger 300 verwendet.

Dieser Objektträger 300 bzw. viele solcher Objektträger 300 kann/können in eine/viele noch nicht kalibrierte Objektträger- bzw. -aufnahme(n) eingesetzt werden. Zur automatischen Kalibrierung kann ein Robotermodul 6 diese in einem Magazin 2 eingesetzten nicht kalibrierten Objektträgerrahmen herausnehmen und auf den Scanningtisch 10 des Scanning-Mikroskop 8 setzen bzw. positionieren.

Das Scanning-Mikroskop 8 detektiert beispielsweise durch Abtasten des Objektträgers 300 die Kalibrierungsmarkierung 304. Die Einstellungen der Messeinrichtung des Scanningtisches 10 bzw. die Scanningtischkoordinaten werden als Markierungskoordinaten erfasst bzw. festgehalten.

In einem nächsten Schritt zum Kalibrieren der Objektträgeraufnahme werden dann neue, nämlich korrigierte, Positionierungskoordinaten der Objektträgeraufnahme festgelegt. Hierzu wird die mit dem vorherigen Verfahren bestimmte Abweichung 308 zu den detektierten Markierungskoordinaten addiert bzw. die Markierungskoordinaten gemäß der Abweichung 308 verändert, so dass spezielle für Objektträgeraufnahme zutreffende Positionierungskoordinaten festgelegt werden.

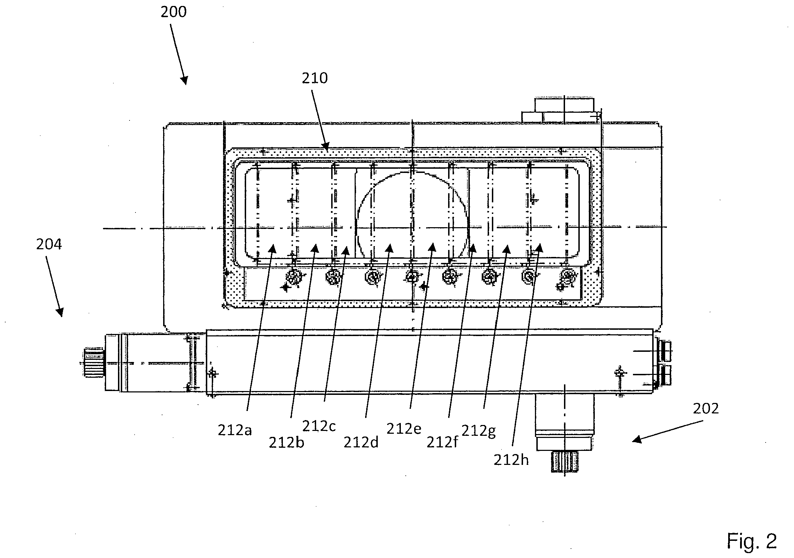

**Figur 2** zeigt schematisch einen Scanningtisch 200 mit einem Objektträgerrahmen 210 und acht eingelegten Objektträgern 212a bis 212h. Der Scanningtisch 200 umfasst weiterhin eine Messeinrichtung bzw. Motoren für eine X-, Y-Verschiebung 202, 204, mit welcher der Scanningtisch 200 eingestellt bzw. verfahren werden kann. Insbesondere kann durch Einstellen der Messeinrichtung 202, 204 der Scanningtisch 200 in eine X-Richtung sowie eine Y-Richtung verfahren werden, so dass bestimmte X- und Y-Koordinaten angefahren werden können. Zusätzlich können Z-Koordinaten mit Hilfe einer Autofokusfunktion eingestellt werden. Beispielsweise können so Objekte, die auf einem Objektträger positioniert sind und denen Objekt-Positionierungskoordinaten zugewiesen wurden, durch Einstellen der X- und Y-Koordinaten sowie ggf. der Z-Koordinaten relokalisiert bzw. angefahren werden.

Anzumerken ist, dass bei der Kalibrierung bzw. beim Erfassen eines Objekts auch die Fokuslage miterfasst werden kann. Dies kann mit Hilfe einer Autofokusfunktion geschehen. Hierdurch kann sichergestellt werden, dass bei einer Relokalisierung eines Objekts dieses einigermaßen "scharf' erscheint (zumindest nahe genug an einer "korrekten" Fokusebene, dass ein Autofokusprogramm mit "relativ kleinem Fangbereich" das Objekt in die "perfekte" Fokusebene bringt".

In einer bevorzugten Ausführungsform werden immer X-, Y- und Z-Koordinaten erfasst.

Insbesondere zeigt **Figur 2** schematisch einen Scanningtisch 200 für 8 Objektträger 212a bis 212h (wie beispielsweise von Märzhäuser, Wetzlar hergestellt) mit einem festem Objektträgerrahmen 210. Dieser Objektträgerrahmen 210 kann beispielsweise die Maße 273 mm x 116 mm aufweisen. Bei einem Wechslersystem mit einem Robotermodul wird anstelle des festen Objektträgerrahmens ein Adapterrahmen eingeschraubt, der drei Zentrierstifte aufweist, auf denen die Wechselrahmen (bspw. Objektträgerrahmen) mit entsprechenden Lagerelementen zu liegen kommen, wobei die Lagerelemente aus einem Fest- und zwei Loslagern (eines mit 2 und eines mit 3 Translationsfreiheitsgraden) bestehen. Solche Lagerungen sind z.B. von justierbaren Spiegelhalterungen bekannt sind. Damit wird eine präzise Reproduzierbarkeit der Objektträgerahmenposition gewährleistet (deutlich unter 5µm).

**Figur 3** zeigt einen Objektträger 300 umfassend ein Objektträgersubstrat 302, eine Kalibrierungsmarkierung 304 und ein Speichermedium 310. Das Objektträgersubstrat 302 kann ein im Laborgebrauch übliches Glas- oder Plastikplättchen sein. Die Kalibrierungsmarkierung 304 kann, wie dargestellt, aus mehreren sich kreuzenden Linien bzw. einem Gitter bestehen.

Alternativ oder zusätzlich kann ein oder mehrere Punkte als Kalibrierungsmarkierung auf bzw. an dem Objektträger 300 vorgesehen sein. Weiterhin können ein Rautenmuster und/oder Kreuze als Kalibrierungsmarkierung vorgesehen sein. Insbesondere sind Kombinationen aus verschiedenen Linien und/oder Punkten bzw. Mustern als Kalibrierungsmarkierung verwendbar, solange die Kalibrierungsmarkierung zuverlässig detektierbar ist und es möglich ist Markierungskoordinaten zu erfassen.

Insbesondere kann als Kalibrierungsmarkierung nur ein Teil bzw. nur bestimmte Punkte der Kalibrierungsmarkierung verwendet werden. Beispielsweise können als Kalibrierungsmarkierung 304 lediglich ein oder mehrere Kreuzungspunkte 306 verwendet werden.

Bei einem kalibrierten Objektträger 300 wurde beispielsweise ein Kreuzungspunkt 306 als Kalibrierungsmarkierung verwendet, um ausgehend von diesem Kreuzungspunkt 306 eine Abweichung 308 zwischen diesem Kreuzungspunkt 306 und einem Mittelpunkt des Objektträgers 300 festzulegen. Die Abweichung 308 ist insbesondere eine Beabstandung von dem Kreuzungspunkt 306, und kann als Δx- sowie Δy-Koordinaten (relative Koordinaten) erfasst werden.

Insbesondere kann als Abweichung 308 auch eine eventuelle Änderungen der Fokuslage erfasst werden, wobei die Änderung der Fokuslage als Az-Koordinaten (relative Koordinaten) erfasst wird.

Diese Abweichung 308 kann beispielsweise in dem Speichermedium 310 hinterlegt sein, so dass diese sofort auslesbar ist. Alternativ kann das Speichermedium 310 als Identifikator des Objektträgers dienen und über ein Auslesen des Speichermediums 310 auf eine Datei zugegriffen werden, in welcher die Abweichung 308 hinterlegt ist. Wie in **Figur 3** dargestellt, kann das Speichermedium 310 ein Barcode bzw. Strichcode sein.

Die Kalibrierungsmarkierung 304 ist beispielsweise zusammen mit dem Barcode auf ein transparentes Trägermaterial 312 gedruckt, wenn der Barcode 310 als Identifikator verwendet wird. Das transparente Trägermaterial 312 kann eine Klebefolie sein.

In anderen Worten wurde ein kostengünstiger Kalibrierslide 300 bzw. Objektträger 300 zum Kalibrieren von Objektträgerrahmen aus einem Objektträgersubstrat 302 hergestellt, indem das Objektträgersubstrat 302 mit einem Etikett 312 mit Linienmuster 304 beklebt wurde. Der Strichcode 310 stellt die Zuordnung des Kalibrierslides zu der Datei mit den Messergebnissen (Abweichung 308 von gemessener Position charakteristischer Kreuzungspunkte zu erwarteter "perfekter" Position) dar.

Somit wird erfindungsgemäß ein Kalibrierslide 300 mit einfachen Mitteln hergestellt, der dann genau vermessen wird.

Zusammengefasst kann die Kalibrierungsmarkierung 304 beispielsweise mit Hilfe eines Laserdruckers auf Etiketten (als beispielhaftes Trägermaterial 312) gedruckt werden. Diese Etiketten werden dann auf entsprechende Objektträgersubstrate 302 geklebt. Danach wird jeder der Objektträger 300 mit einer Kalibrierungsmarkierung 304 (Kalibrierslide) zunächst individuell vermessen. Dies geschieht vollautomatisch mit Hilfe eines bereits kalibrierten Objektträgerrahmens bzw. Wechslersystems. Die für die Kalibrierung verwendeten Linienkreuzungen bzw. Kreuze bzw. Punkte, etc. werden dabei automatisch detektiert, und ihre Koordinaten werden in einer individuellen Kalibrierdatei abgelegt. Nach der Kalibrierung eines jeden Objektträgers 300 (Kalibrierslides) wird ein Barcode 310 aufgebracht, der die gemessene Abweichung 308 bzw. Abweichungskoordinaten von den markierten Positionen (Kalibrierungsmarkierung) enthält bzw. auf die Datei verweist, die diese Daten enthält.

Insbesondere können die Etiketten transparent oder nicht-transparent sein, wobei der Begriff "nicht-transparent" derart zu verstehen ist, dass das nicht-transparente Etikett für Umgebungslicht (natürliches Licht bzw. sichtbares Licht) nicht durchlässig ist bzw. nur in "geringem Maße" durchlässig ist. Insbesondere kann Umgebungslicht (ggf. von einer Schreibtischleuchte abgestrahlt) in Reflexion bezüglich des nichttransparenten Etiketts verwendet werden.

Bei der Neukalibrierung von Objektträgerrahmen bzw. eines Wechslersystems werden alle Objektträgeraufnahmen (Kalibrierslidepositionen) - beispielsweise pro Magazin 16 Objektträgerrahmen à 5 Objektträgeraufnahmen (Slidepositionen) - mit zuvor kalibrierten Objektträgern 300 (vermessenen Kalibrierslides) bestückt. In der nun folgenden automatischen Kalibrierung findet die Software die Kreuzungspunkte 304, 306 auf jedem Objektträger 300 (Kalibrierslide) und korrigiert die aktuelle Position des Scanningtisches 10 mit der bekannten Abweichung 308, die in der zugehörigen Kalibrierdatei des Objektträgers 300 (Kalibrierslides) abgelegt ist. Dabei erfolgt die eindeutige Zuordnung von Objektträger 300 (Kalibrierslide) zu Kalibrierdatei über das Auslesen des auf jedem Objektträger 300 (Kalibrierslide) aufgebrachten Barcodes 310. Zusätzlich wird jeder Objektträgerrahmen über einen eigenen Aufkleber mit einem Barcode identifiziert, da erst die Nummer des Objektträgerrahmens und die Nummer der Objektträgeraufnahme die individuelle absolute Objektträgerposition eines Objektträgers in diesem Objektträgerrahmen definieren. Die resultierenden Kalibrierdaten werden für alle (bspw. bis zu 880 möglichen) Objektträgeraufnahmen bzw. Positionen eines Objektträgers in dem Magazin gespeichert.

### Bezugszeichenliste

- 100: Mikroskop-Scanningsystem
- 2: Magazin
- 4,210: Objektträgerrahmen
- 6: Robotermodul
- 8: Scanning-Mikroskop 8
- 10, 200: Scanningtisch
- 202, 204: Messeinrichtung / Motoren für X-,Y-Verschiebung
- 212a -212h, 300: Objektträger
- 302: Objektträgersubstrat
- 304: Kalibrierungsmarkierung
- 306: Kreuzungspunkt
- 308: Abweichung
- 310: Speichermedium
- 312: transparente Trägermaterial

## Patentansprüche

1. Verfahren zur automatischen Kalibrierung von Objektträgern (300) umfassend die Schritte:
- Bereitstellen eines Objektträgerrahmens (4; 210) mit mehreren Objektträgeraufnahmen, wobei der Objektträgerrahmen kalibriert ist und dadurch die Positionierungskoordinaten der Objektträgeraufnahmen bezüglich eines Mikroskop-Scanningsystems (100) festgelegt sind, und wobei die Positionierungskoordinaten in einem Speichermedium abgespeichert sind, welches von dem Mikroskop-Scanningsystem (100) auslesbar ist;
- Bereitstellen zumindest eines Objektträgers (212a - 212h; 300) mit einer Kalibrierungsmarkierung (304);
- Anordnen des zumindest einen Objektträgers (212a - 212h; 300) in einer derObjektträgeraufnahmen des Objektträgerrahmens (4; 210);
- Anordnen des Objektträgerrahmens mit dem Objektträger (212a - 212h; 300) auf einem Scanningtisch (10; 200) des Mikroskop-Scanningsystems (100);
- Erfassen von Markierungskoordinaten der Kalibrierungsmarkierung (304) basierend auf einer Detektion der Kalibrierungsmarkierung (304) des Objektträgers (212a -212h; 300) durch das Mikroskop-Scanningsystem (100);
- Erfassen einer Abweichung (308) zwischen den Positionierungskoordinaten der Objektträgeraufnahme und den Markierungskoordinaten des Objektträgers (212a -212h; 300); und
- Hinterlegen der erfassten Abweichung (308) in einem Speichermedium (310), welches mit dem Objektträger (212a - 212h; 300) verbindbar ist.

2. Verfahren nach Anspruch 1, wobei die Abweichung (308) eine oder mehrere Abweichungskoordinate(n) umfasst.

3. Verfahren nach Anspruch 2, wobei die Abweichungskoordinaten einen maximalen abtastbaren Scanbereich des Objektträgers (212a - 212h; 300) festlegen.

4. Verfahren nach Anspruch 2 oder 3, wobei die Abweichungskoordinaten einen Mittelpunkt des Objektträgers (212a - 212h; 300) festlegen.

5. Verfahren nach einem der Ansprüche 2 bis 4, wobei die Abweichungskoordinaten zumindest zwei Referenzpunkte festlegen, die eine Transformation von Koordinaten der Objekträgeraufnahme im Bezug zu Koordinaten des Scanningtisches (10; 200) des Mikroskop-Scanningsystems festlegen.

6. Verfahren nach Anspruch 1, wobei das Speichermedium (310) ein Barcode oder ein RFID-Etikett ist.

7. Verfahren nach einem oder mehreren der vorangehenden Ansprüche, wobei die Kalibrierungsmarkierung (304) eine oder mehrere Linien und/oder Punkte umfasst.

8. Verfahren nach einem oder mehreren der vorangehenden Ansprüche, wobei die Kalibrierungsmarkierung (304) auf einem transparenten Trägermaterial aufgebracht ist, welches lösbar mit dem Objektträger (212a - 212h; 300) verbindbar ist.

9. Verfahren nach Anspruch 8, wobei das transparente Trägermaterial eine Klebefolie ist.

10. Verfahren nach einem oder mehreren der vorangehenden Ansprüche, wobei die Kalibrierungsmarkierung (304) durch Bedrucken des Objektträgers (212a - 212h; 300) und/oder des transparenten Trägermaterial (312) aufgebracht wird.

11. Verfahren nach einem oder mehreren der vorangehenden Ansprüche, wobei der Objektträgerrahmen (4; 210) eine Vielzahl von Objektträgeraufnahmen umfasst, und in jede Objektträgeraufnahme jeweils ein Objektträger (212a - 212h; 300) mit einer Kalibrierungsmarkierung (304) eingesetzt wird, um für jeden Objektträger (212a - 212h; 300) eine Abweichung (308) zu erfassen.

## Claims

1. A method for the automatic calibration of slides (300) comprising the steps:
- Providing a slide frame (4; 210) having a plurality of slide seats, wherein the slide frame is calibrated which establishes the positioning coordinates of the slide seats with respect to a microscope scanning system (100), and wherein the positioning coordinates are saved in a storage medium that can be read out by the microscope scanning system (100);
- Providing at least one slide (212a - 212h; 300) with a calibration mark (304);
- Arranging the at least one slide (212a - 212h; 300) in one of the slide seats of the slide frame (4; 210);
- Arranging the slide frame with the slide (212a - 212h; 300) on a scanning table (10; 200) of the microscope scanning system (100);
- Detecting mark coordinates of the calibration mark (304) based on a detection of the calibration mark (304) of the slide (212a -212h; 300) by the microscope scanning system (100);
- Detecting a deviation (308) between the position coordinates of the slide seat and the mark coordinates of the slide (212a -212h; 300); and
- Saving the detected deviation (308) in a storage medium (310) that can be connected to the slide (212a - 212h; 300).

2. The method according to claim 1, wherein the deviation (308) comprises one or more deviation coordinate(s).

3. The method according to claim 2, wherein the deviation coordinates establish a maximum scannable scanning range of the slide (212a - 212h; 300).

4. The method according to claim 2 or 3, wherein the deviation coordinates establish a

5. The method according to one of claims 2 to 4, wherein the deviation coordinates establish at least two reference points that establish a transformation of coordinates of the slide seat with respect to the coordinates of the scanning table (10; 200) of the microscope scanning system.

6. The method according to claim 1, wherein the storage medium (310) is a barcode or an RFID label.

7. The method according to one or more of the preceding claims, wherein the calibration mark (304) comprises one or more lines and/or points.

8. The method according to one or more of the preceding claims, wherein
the calibration mark (304) is applied to a transparent carrier material that is releasably connectable to the slide (212a - 212h; 300).

9. The method according to claim 8, wherein the transparent carrier material is an adhesive film.

10. The method according to one or more of the preceding claims, wherein the calibration mark (304) is applied by printing the slide (212a - 212h; 300) and/or the transparent carrier material (312).

11. The method according to one or more of the preceding claims, wherein the slide frame (4; 210) comprises a plurality of slide seats, and a slide (212a - 212h; 300) with a calibration mark (304) is inserted into each slide seat in order to detect a deviation (308) for each slide (212a - 212h; 300).

## Revendications

1. Procédé d'étalonnage automatique de lames (300) comportant les étapes suivantes :
- mise à disposition d'un cadre de lame (4 ; 210) avec plusieurs logements de lame, le cadre de lame étant étalonné et les coordonnées de position des logements de lame par rapport à un microscope à balayage (100) étant ainsi définies, et les coordonnées de position étant enregistrées dans un support de stockage susceptible d'être lu par le microscope à balayage (100) ;
- mise à disposition d'au moins une lame (212a - 212h ; 300) avec un marqueur d'étalonnage (304) ;
- agencement de l'au moins une lame (212a - 212h ; 300) dans l'un des logements de lame du cadre de lame (4 ; 210) ;
- agencement du cadre de lame avec la lame (212a - 212h ; 300) sur une table de balayage (10 ; 200) du microscope à balayage (100) ;
- détection des coordonnées de marqueur du marqueur d'étalonnage (304) sur la base d'une détection du marqueur d'étalonnage (304) de la lame (212a - 212h ; 300) par le microscope à balayage (100) ;
- détection d'un écart (308) entre les coordonnées de position du logement de lame et les coordonnées de marqueur de la lame (212a - 212h ; 300) ; et
- mémorisation de l'écart (308) détecté dans un support de stockage (310) susceptible d'être relié à la lame (212a - 212h ; 300).

2. Procédé selon la revendication 1, dans lequel l'écart (308) comporte une ou plusieurs coordonnée(s) d'écart.

3. Procédé selon la revendication 2, dans lequel les coordonnées d'écart fixent une zone de balayage maximale explorable de la lame (212a - 212h ; 300).

4. Procédé selon la revendication 2 ou 3, dans lequel les coordonnées d'écart fixent un point central de la lame (212a - 212h ; 300).

5. Procédé selon l'une des revendications 2 à 4, dans lequel les coordonnées d'écart fixent au moins deux points de référence, lesquels fixent une transformation de coordonnées du logement de lame par rapport aux coordonnées de la table de balayage (10 ; 200) du microscope à balayage.

6. Procédé selon la revendication 1, dans lequel le support de stockage (310) est un code-barres ou une étiquette RFID.

7. Procédé selon l'une ou plusieurs des revendications précédentes, dans lequel le marqueur d'étalonnage (304) comporte une ou plusieurs lignes et/ou un ou plusieurs points.

8. Procédé selon l'une ou plusieurs des revendications précédentes, dans lequel le marqueur d'étalonnage (304) est appliqué sur un matériau de support transparent, lequel peut être relié de façon amovible à la lame (212a - 212h ; 300).

9. Procédé selon la revendication 8, dans lequel le matériau de support transparent est une feuille adhésive.

10. Procédé selon l'une ou plusieurs des revendications précédentes, dans lequel le marqueur d'étalonnage (304) est appliqué par impression sur la lame (212a - 212h ; 300) et/ou le matériau de support transparent (312).

11. Procédé selon l'une ou plusieurs des revendications précédentes, dans lequel le cadre de lame (4 ; 210) comporte une pluralité de logements de lame, et une lame (212a - 212h; 300) avec un marqueur d'étalonnage (304) est introduite respectivement dans chaque logement de lame, pour détecter un écart (308) pour chaque lame (212a - 212h ; 300).
